# EUROPEAN PATENT SPECIFICATION

(11) **EP 1 233 987 B1**
(45) Date of publication and mention of the grant of the patent: **19.08.2009**
(21) Application number: 00985080.1
(22) Date of filing: 22.11.2000
(51) Int. Cl.: C07K 17/00, C07K 1/22, G01N 33/543, G01N 33/563, C07K 16/00

(54) **IMMOBILIZED SINGLE DOMAIN ANTIGEN-BINDING MOLECULES**
IMMOBILISIERTE ANTIGENBINDENDE MOLEKÜLE AUS EINER DOMÄNE
IMMOBILISATION DE MOLECULES DE LIAISON D'ANTIGENE A DOMAINE UNIQUE

(30) Priority: 29.11.1999 EP 99309516
(43) Date of publication of application: 28.08.2002
(73) Proprietor: BAC IP B.V., 1411 GP Naarden (NL)
(72) Inventor: FRENKEN, Leo G. J., Unilever Research V, 3130 AC Vlaardingen (NL); GRANT, Steven Daryl, Unilever Research Colworth, Bedford, Bedfordshire MK44 1LQ (GB); HAAFT, Ten Mark R., Unilever Research Vlaardingen, 3133 AT Vlaardingen (NL); VAN DER LOGT, Cornelis, P., Unilever Res. Vlaard., 3133 AT Vlaardingen (NL)
(74) Representative: Swinkels, Bart Willem
(86) International application number: PCT/EP2000/011656
(87) International publication number: WO 2001/044301

(56) References cited:
- EP-A- 0 434 317
- EP-A- 0 584 421
- WO-A-91/08482
- WO-A-99/23221
- M. LAUWEREYS ET AL.: "Potent enzyme inhibitors derived from dromedary heavy-chain antibodies." THE EMBO JOURNAL, vol. 17, no. 13, 1 July 1998 (1998-07-01), pages 3512-3520, XP002136362 Oxford, GB
- M. ARBABI-GHAHROUDI ET AL.: "Selection and identification of single domain antibody fragments from camel heavy-chain antibodies." FEBS LETTERS, vol. 414, no. 3, 15 September 1997 (1997-09-15), pages 521-526, XP002069903 Amsterdam, The Netherlands
- J. DAVIES ET AL.: "Single antibody domains as small recognition units: design and in vitro antigen selection of camelized, human VH domains with improved protein stability." PROTEIN ENGINEERING, vol. 9, no. 6, June 1996 (1996-06), pages 531-537, XP002080377 Oxford, GB
- BERRY M.; DAVIES J.: 'Use of antibody fragments in immunoaffinity chromatography.' JOURNAL OF CHROMATOGRAPHY vol. 597, 1992, THE NETHERLANDS, pages 239 - 245

## Description

### FIELD OF THE INVENTION

The present invention relates to the immobilisation of the antigen binding domains (VHH) of antibodies naturally devoid of light chains or functional equivalents thereof at solid surfaces with retention of binding activity. More particularly, the invention relates to the preparation and use of immunoactive materials comprising a VHH domain, without any peptide extensions, immobilised on a solid surface by means of covalent interactions.

### BACKGROUND OF THE INVENTION

Processes directed towards immobilising proteins on to a solid surface are of considerable commercial interest. Functionalisation of surfaces with immunological materials, such as antibodies or antibody fragments, in particular, forms the basis of immunoadsorption techniques such as immuno-affinity purification processes which are increasingly being applied to the recovery or purification of a range of commercially important materials.

Commonly, attachment of proteins to solid surfaces, such as chromatography media, has been brought about by exposing the surface to a solution of the protein such that the protein is adsorbed onto the solid surface via non-specific binding mechanisms. Methods for immobilising proteins on chromatography media are well established in the literature, see for example, in Protein Immobilisation, R.F. Taylor ed., Marcel Dekker, Inc., New York, 1991. Where the solid surface is provided by a hydrophobic material such as polystyrene, for example, then attachment is generally brought about by adsorption of hydrophobic regions of the protein onto the hydrophobic surface.

Adsorption onto the solid surface is usually accompanied by significant conformational disruption with partial unfolding and denaturation of the protein concerned. The concomitant loss of protein activity detracts from the overall usefulness of the process. Commonly, for example, adsorption of antibodies onto a hydrophobic surface is accompanied by the loss of in the order of greater than 95% of binding activity as well as diminished binding specificity. Where smaller antibody fragments are involved, the degree of specific binding affinity retained upon adsorption onto a solid surface can be even lower, as described by Molina-Bolivar et al, J. Biomaterials Science-Polymer Edition, 9, 1103-1113, 1998.

Alternatives to, or improvements upon, the method of adsorption of proteins in the preparation of immobilised protein surfaces have been considered.

One alternative approach is to use chemical cross-linking of residues in the protein for covalent attachment to an activated solid surface using conventional coupling chemistries, for example as described in Bioconjugate Techniques, G.T. Hermanson, ed. Academic Press, Inc., San Diego, CA, USA. Amino acid residues incorporating sulphydryl groups, such as cysteine, may be covalently attached using a bispecific reagent such as succinimidyl-maleimidophenylbutyrate (SMPB), for example. Alternatively, lysine groups located at the protein surface may be coupled to activated carboxyl groups at the solid surface by conventional carbodiimide coupling using 1, ethyl-3-[3-dimethyl aminopropyl] carbodiimide (EDC) and N-hydroxysuccinimide (NHS). A disadvantage of this approach is that cross-linking residues in the protein can interfere with the functionality of the protein. The smaller the antibody fragment to be immobilised, the greater the probability that cross-linking will involve a residue in, or close to, the antigen binding site and, consequently, interfere with binding activity. This has been noted specifically for the case of isolated classical VH domains, where cross-linking was found to lead to substantial losses in binding affinity and specificity (see, for example, Berry et al, Journal of Chromatography, 597, 239-245 (1992), which suggests that classical VH domains are more readily inactivated than Fv fragments during immobilisation). As shown by Spitznagel et al, Bio/Technology, 11, 825-829, (1993), in addition to decreased specific activities, immobilised antibodies typically exhibit lower binding affinities than their soluble counterparts.

By providing the antibody fragment with an extension in the form of a peptide tail or an additional protein domain, attachment of the protein to the surface can be brought about by non-covalent adsorption or using conventional chemical cross-linking agents at a site remote from the main body of the protein. In this way, the immobilisation process itself is less likely to interfere with the functionality of the protein.

Rational coupling procedures to control the orientation of the antibody fragment can also be employed (Rao et al, Mikrochimica Acta, 128 (3-4), 127-143, 1998). Furthermore, increasing the length of the chemical linker used to attach the immobilised molecule to the solid support can reduce the conformational stresses on the protein, allowing it to exist in a more natural conformation.

EP 0434317 (Joseph Crosfield & Sons) discloses the use of improved affinity purification media which employ small specific binding agents, especially Fv antibody fragments. These optionally have a hydrophobic tail, with a particularly preferred linking group being the myc epitope (Munro, S., and Pelham, H.R. (1986) Cell 46, 291-300). Such a tail is primarily intended to facilitate immobilisation of the binding agent by non-covalent attachment onto a hydrophobic surface although as the myc group contains a lysine residue, it could also be used for covalent attachment onto surfaces.

WO 91/08482 (Unilever) describes in Example 6 an anti-lysozyme VH with no tail, immobilised on a solid phase; from the results presented, it is clear that antibody binding activity is adversely and severely affected compared to VH fragments immobilised at a solid surface via a peptide tail.

There remains a continuing need to improve the ability to prepare immunoactive surfaces by immobilisation of antibody fragments with retention of their binding affinity and specificity, preferably without the need to elaborate them with tail sequences.

A class of antibodies of particular interest in relation to biotechnological applications comprises the Heavy Chain antibodies such as found in camelidae, like the camel or the llama. The binding elements of these antibodies consist of a single polypeptide domain, namely the variable region of the heavy chain polypeptide (VHH). These antibodies are naturally devoid of light-chains with the heavy chain variable domain forming the complete antigen-binding site. In contrast, classical antibodies (murine, human, and so on), have binding elements comprising two polypeptide domains (the variable regions of the heavy chain (VH) and the light chain (VL)). The lack of dependence on interaction with a light chain variable domain for maintaining structural and functional integrity gives these VHH domains a substantial advantage over other small antibody fragments, in terms of ease of production and behaviour in solution. In particular, VHH fragments are the preferred types of molecules for immuno-affinity purification, because of their unusual stability and their ability to refold efficiently after complete denaturation, which frequently occurs during elution of antigen.

M.Arbabi-Ghahroudi et al, FEBS Letters, vol 414 no 3, 15 September 1997, pages 521-526 discloses the selection and identification of single domain antibody fragments from camel heavy-chain antibodies (VHH domain).

Procedures to obtain heavy chain immunoglobulins from Camelidae, or (functionalised) fragments thereof, have been described in WO 94/04678 (Casterman et al) and WO 94/25591 (Frenken et al). VHH fragments can be produced through recombinant DNA technology in a number of microbial hosts (bacterial, yeast, mould), as described in WO 94/29457 (Frenken et al). Alternatively, binding domains can be obtained by modification of the VH fragments of classical antibodies by a procedure termed "camelisation", described by Davies et al, Bio/Technology, 13, 475-479 (1995). Hereby the classical VH fragment is mutated, by substitution of a number of amino acids present in the VH/VL interface, into a VHH-like fragment, whereby its binding properties are retained.

The immobilisation of a VHH fragment with a His-tail on a carboxylated dextran layer using EDC/NHS coupling chemistry is described by Muyldermans et al, EMBO Journal, 17 (13), p3512-3520, 1998. Polypeptide tail-mediated immobilisation of VHH fragments on solid surfaces is also proposed in WO 99/23221 (Unilever).

### SUMMARY OF THE INVENTION

The invention describes the immobilisation of single-domain antigen-binding fragments from antibodies naturally devoid of light chains (VHH), or protein domains functionally equivalent thereto, onto a solid surface through covalent cross-links while still retaining sufficient biological activity to enable the fragments to function (e.g. as in an affinity purification matrix). The fragments themselves are immobilised directly on to the solid surface; they are devoid of any appended polypeptide group through which immobilisation could otherwise be mediated. The VHH fragments described are typically less than 20 kDa in molecular weight and contain only the amino acid sequence necessary to form a complete functional VHH fragment or a proportion thereof (truncated fragment).

The present invention may be more fully understood with reference to the following description when read together with the accompanying drawings in which:
Figure 1 shows an analysis by SDS-PAGE of the coupling of VHH antibody fragments to CNBR sepharose 4B (Amersham Pharmacia Biotech). The proteins were stained using Coomassie brillaint blue. The molecular weight marker set was from GibcoBRL (BenchMark Lot. No.: 1080925).
Figure 2 shows chromatograms obtained during purification of mouse IgG from serum by immuno-affinity chromatography using the immobilised tagged version (coded 1A) and the untagged version (coded 3) of VHH#1.
Figure 3 shows samples from purification of mouse-IgG from serum by immuno-affinity chromatography, analysed on a Coomassie brilliant blue-stained SDS-PAGE gel. Left panel: purification using immobilised, untagged VHH#1. (lane 1: molecular weight markers; lane 2: mouse serum; lane 3: flow through (fraction 3 - see figure 2); lane 4: elution (fraction 10 - see figure 2).
   Right panel: purification using immobilised, tagged VHH#1. (lane 1: molecular weight markers; lane 2: mouse serum; lane 3: flow through (fraction 3); lane 4: elution (fraction 10).
   Molecular weight markers: Marker GibcoBRL BenchMark (Lot. No.: 1080925)

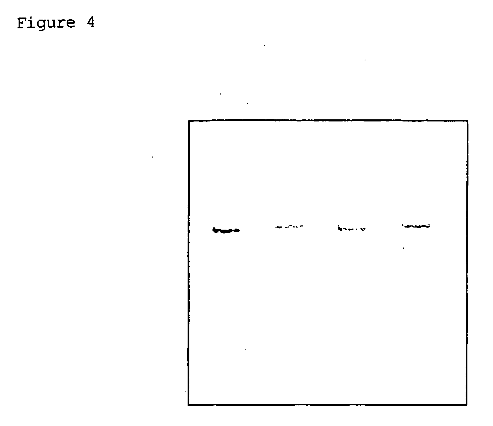

Figure 4 shows samples from purification of IgG from mouse serum analysed on Western blot.
Lane 1,5,9: markers
Lanes 2-4: purification using immobilised, untagged VHH#1 (lane 2: mouse serum; lane 3: flow through (fraction 3 - see figure 2); lane 4: elution (fraction 10 - see figure 2).
Lanes 6-8: purification using immobilised, tagged VHH#1 (lane 6: mouse serum; lane 7: flow through (fraction 3 - see figure 2); lane 8: elution (fraction 10 - see figure 2)
Molecular weight markers: Marker GibcoBRL BenchMark (Lot. No.: 1080925).

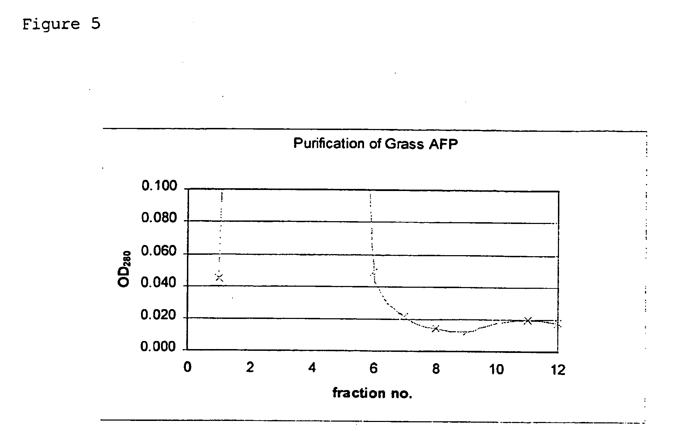

Figure 5 shows a chromatogram obtained during purification of grass AFP from broth by immuno-affinity chromatography using the immobilised, untagged version of VHH#2.

### DETAILED DESCRIPTION OF THE INVENTION

The invention is based on the finding that cloned VHH fragments can be attached to a solid phase matrix surface (and still retain sufficient activity to function as the affinity ligand of a purification matrix) in the absence of any appended polypeptide group such as peptide tails or extra (non-variable heavy domain) protein domains.

This contrasts surprisingly with the situation with classical VH fragments where, in order to retain binding activity, it has been found to be necessary to provide the fragment with a polypeptide tail through which immobilisation can be mediated, such that attachment to the solid surface occurs at a location remote from the antigen binding site. The conventional rationale for this is that all residues in such a small binding domain are likely to be important either for antigen binding or for ensuring structural integrity and therefore that cross-linking at any point in the molecule will tend to result in its inactivation. Given that VHH and VH domains are similar in size, it would generally have been expected that they both would be subject to similar inactivating perturbations upon immobilisation at a surface.

The present inventors have clearly shown that immobilisation of VHH domains, devoid of any peptide extensions, at a surface through covalent cross-links does not destroy their ability to bind antigen with high affinity and selectivity. Further, it has been shown that provision of a cross-linkable peptide extension improves neither the efficiency of the cross-linking reaction nor the antigen-binding capacity or selectivity of the bound VHH domains.

The ability to attach functional antibody fragments directly to a surface, rather than via a fused tag sequence, is extremely advantageous because it is well-known that such fusion proteins are highly susceptible to proteolysis, with consequent loss of the immobilised antibody from the surface; see for example McCafferty, J. et al (1990) Nature 348, 552-554.

A VHH domain is a heavy chain variable domain derived from an immunoglobulin naturally devoid of light chains, such as may be obtained from camelids as described above. The antigen-binding capacity and specificity is located naturally and exclusively in the heavy chain variable domain; that is, the heavy chain variable domain forms the complete antigen-binding site.

The terms "immunoglobulin" and "antibody" are used synonymously throughout this specification unless indicated otherwise.

An advantage of using immunoglobulins or heavy chain variable domains naturally devoid of light chains is that they can be readily and conveniently produced economically on a large scale, for example, using a transformed lower eukaryotic host as described in WO 94/25591, mentioned above. Further, as mentioned above, the absence of dependence on a light chain for ensuring structural and functional integrity renders isolated VHH domains more stable and easier to handle than other small antibody fragments such as classical VH domains. This is especially advantageous for the preparation of reusable affinity matrices.

By functionally equivalent is meant any protein which has the same or similar antigen binding properties located in a single binding domain. It will be appreciated that proteins modified to enable them to function as binding domains in the same way as heavy chain immunoglobulin variable domains from camelids (see Davies et al, Bio Technology, 13, 475-479, (1995)) may also suitably be used according to the invention.

According to one embodiment of the invention, the VHH domain or functionally equivalent protein is attached to a solid surface by covalent cross-linking using conventional coupling chemistries.

It will be appreciated that since the VHH domain or functional equivalent is attached to a solid surface by covalent coupling, the surface must be capable of being covalently coupled to the VHH domain. The solid surface may naturally comprise cross-linkable residues suitable for covalent attachment or it may be coated or derivatised to introduce suitable cross-linkable groups according to methods well known in the art.

The solid surface onto which immobilisation according to the invention takes place may be provided by a variety of materials and may suitably be any solid phase carrier material conventionally used in immobilising proteins.

The invention is applicable to any solid phase material that is amenable to the immobilisation of proteins or protein fragments, either directly or after pre-treatment. The carrier materials may be particulate (e.g. beads or granules, generally used in extraction columns) or in sheet form (e.g. membranes or filters, glass or plastic slides, microtitre assay plates, dipstick, capillary fill devices or such like) which can be flat, pleated, or hollow fibres or tubes. The following matrices are given as examples and are not exhaustive, such examples could include silica (porous amorphous silica), i.e. the FLASH series of cartridges containing 60A irregular silica (32-63 µm or 35-70 µm) supplied by Biotage (a division of Dyax Corp.), agarose or polyacrylamide supports, for example the Sepharose range of products supplied by Amersham Pharmacia Biotech, or the Affi-Gel supports supplied by Bio-Rad. In addition there are macroporous polymers, such as the pressure-stable Affi-Prep supports as supplied by Bio-Rad. Other supports that could be utilised include; dextran, collagen, polystyrene, methacrylate, calcium alginate, controlled pore glass, aluminium, titanium and porous ceramics. Alternatively, the solid surface may comprise part of a mass dependent sensor, for example, a surface plasmon resonance detector. Further examples of commercially available supports are discussed by Taylor, R.F. (1991), referred to above. Conveniently, an array comprising plurality of individual antigen-binding fragments bound to a solid surface is provided.

Advances in molecular biology, particularly through site- directed mutagenesis, enable the mutation of specific amino acid residues in a protein sequence. The mutation of a particular amino acid (in a protein with known or inferred structure) to a lysine or cysteine (or other desired amino acid) can provide a specific site for covalent coupling, for example. It is also possible to reengineer a specific protein to alter the distribution of surface available amino acids involved in the chemical coupling (Kallwass et al, Biotechnol. Lett., 15 (1), 29-34, 1993), in effect controlling the orientation of the coupled protein. A similar approach can be applied to antibody fragments, specifically VHH fragments, so providing a means of oriented immobilisation without the addition of extra-VHH peptide tails or domains containing either natural or unnatural amino acids. Introduction of mutations in the framework region of the antibody fragment is preferred, minimising disruption to the antigen-binding activity of the VHH fragment. A particular region of the VHH fragment suitable for mutagenesis is in the portion of the molecule that is in proximity to the constant heavy domain, which occurs in naturally produced heavy chain antibodies.

A particular advantage of the invention is that sufficient biological activity of the antibody domain, more specifically the VHH fragment, is retained following immobilisation to allow the coupled fragment to function as an affinity ligand. In one particular embodiment, sufficient functionality is retained following direct covalent coupling to the desired matrix via a reactive moiety that does not contain a chemical spacer arm.

Materials prepared according to the invention may be used in any process where it is useful to bind a molecule to an immobilised antibody fragment. Suitable applications will readily suggest themselves to the average skilled person in the art. Conveniently, the immobilised materials may be used in immunoadsorption processes such as immunoassays, for example ELISA, or immunoaffinity purification processes by contacting a material according to the invention with a test sample according to standard methods conventional in the art. Alternatively, an assay comprising a plurality of individual antigen-binding fragments bound to a solid surface according to the invention may be used, for example, to test for presence of one or more specific binding partners.

### EXAMPLES

The following examples are provided by way of illustration only, are not exhaustive and should not be considered as limiting the scope of the invention. Techniques used for the manipulation and analysis of nucleic acid materials can be performed as described in Sambrook et al, (1989), unless otherwise indicated.

### EXAMPLE 1: Isolation of Anti-mouse Fc and Anti-Anti Freeze Peptide VHH fragments.

The following protocol is taken as an example of how specific VHH fragments can be isolated, cloned, expressed, then coupled onto the desired matrix:

Although the particular VHH fragments described in this example were derived from an immune repertoire, they also could have been selected from a synthetic/semi-synthetic naive VHH library (see WO 00/43507, Unilever).

A llama was immunised with Fc fragments prepared from polyclonal IgG's from mouse serum or with an Anti-Freeze Peptide (AFP) from *Lolium perenne* (grass AFP) (Sidebottom et al. (2000), Nature 406, 256). The llama was boosted several times following the initial immunisation (one month between injections) to increase the specificity of the immune response. DNA encoding specific VHH fragments can then be isolated using similar methods to those described in WO 94/04678 (Casterman et al). If required an immune repertiore of VHH fragments can be selected against the desired antigen as described in WO 94/18330 (Frenken et al). Alternatively selection methods based on phage display can be used to isolate the anti-Fc VHH or anti-AFP VHH producing clones from immune repertoires.

The encoded amino acid sequence of the isolated anti-Fc VHH fragment used in this example is presented below:

### VHH#l

(cloned in *E. coli* as a MYC-His6-tagged VHH in production plasmid pUR1490 and as untagged VHH in production plasmid pUR1491):

The amino acid sequence of the anti-AFP VHH used in this example is as follows:

### VHH#2

(cloned in *E. coli* as a MYC-His6-tagged VHH in production plasmid pUR1492 and as untagged VHH in production plasmid pUR1493):

The tags used are C-MYC (shown in bold below), recognized by monoclonal antibody 9E10 (Munro, S., and Pelham, H.R. (1986) Cell 46, 291-300), followed by a 12-mer peptide encoding an *in vivo* biotinylation signal (shown in bold and underlined) and the hexahistidine tail (shown in italics) for purification with IMAC (Hochuli, E., Bannwarth, W., Döbeli, H., Gentz, R., and Stüber, D. (1988) Biotechnology 6, 1321-1325); the complete sequence fused to the carboxy-terminus of the VHH is presented below:

### EQKLISEEDLN GAA LRSIFEAQKMEW HHHHHH

### EXAMPLE 2: Production and purification of VHH fragments.

For expression in *Escherichia coli* using the production plasmids indicated in example 1, transformants were cultivated in 400 ml 2TY medium and induced with isopropyl-β-D-thiogalactopyranoside as described (Skerra and Pluckthun (1991) Prot. Eng. 4, 971-979). Cells were harvested by centrifugation and lysed in the French Press. Insoluble protein was removed by centrifugation and from the soluble protein fraction the tagged VHH was purified.

Isolated VHH fragments may alternatively be expressed in a transformed lower eukaryote such as *Saccharomyces cerevisiae* or *Pichia pastoris* as described in WO 94/25591 (Frenken et al). Tagged VHH may be purified by IMAC using TALON column material according to the instructions of the supplier (Clontech). Expressed VHH fragments without tags may be isolated by either Protein-G or Protein-A affinity chromatography for example using HiTrap rProtein-A columns (Pharmacia Biotech) according to the manufacturer's instructions.

Alternatively, if the VHH fragments cannot be purified by Protein-G or Protein-A then ion exchange chromatography may be used as an alternative, for example using an AKTAexplorer chromatography system (Pharmacia Biotech) according to the manufactures' instructions. Otherwise matrices containing coupled antigen could be used to purify the fragments.

Purification of the non-tagged VHH antibody fragments (anti-grass AFP and anti-mouse Fc) was performed using a Centriplus-50 spin concentrator (Amicon); the concentrator was centrifuged at 3,000 g (Sorvall RCSB). Purification of the tagged anti-Mouse Fc VHH fragment was performed with IMAC using TALON column material according to the instructions of the supplier (Clontech).

### EXAMPLE 3: Coupling of VHH to CNBr activated Sepharose 4B.

Between 200 µg and 2 mg of VHH fragment was coupled to CNBr Sepharose 4B (approx. 0.3 g, Amersham Pharmacia Biotech, Product code 17-0430-01) following the manufacturer's instructions. Prior to coupling a buffer exchange was performed by gel filtration on PD10 columns columns (Amersham Pharmacia Biotech), so that the VHH fragments would be in a buffer compatible with the immobilisation procedure. The coupled material was packed into a 1 cm diameter column, to give a bed volume of approximately 1.5 ml. The column was then washed with buffer A (10 mM Na2HPO4/NaH2PO4 150 mM NaCl pH 7.4) and pre-eluted with elution buffer B (10 mM Na2HPO4/NaH2PO4 150 mM NaCl pH 2.1), then washed with approximately 5 column volumes buffer A.

Four different fragments were coupled to a CNBr activated matrix in this way, the anti-mouse Fc VHH#1 and the anti-grass AFP VHH#2; both the tagged and the untagged versions.

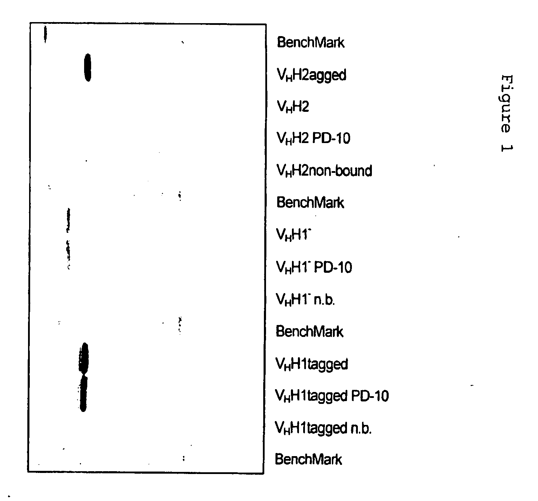

Analysis of the fractions before and after coupling on a Coomassie stained gel showed that the degree of coupling of the tagged as well as the untagged version of the two different VHH fragments was high and revealed no difference in coupling efficiency between the tagged and untagged VHH (see Fig. 1).

This result demonstrates that VHH fragments can be efficiently coupled to a solid surface via covalent bonds even when no peptide tail is present to provide additional sites for cross-linking.

### EXAMPLE 4: Purification of monoclonal and polyclonal IgG's with the anti-Fc VHH affinity columns.

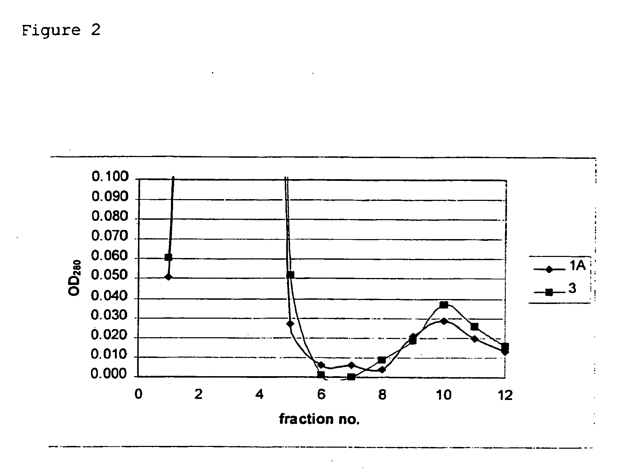

To analyse the performance of the matrices prepared according to example 3, several purifications were performed. After coupling of both the tagged and untagged Anti-mouse Fc VHH, complete mouse serum was loaded onto the columns. The buffers for the purification were, buffer A: 10 mM Na2HPO4/NaH2PO4 150 mM NaCl pH 7.4, and elution buffer B 10 mM Na2HPO4/NaH2PO4 150 mM NaCl with an addition of 12 mM HCl, final pH 2. Fraction collection was done manually, fractions were immediately neutralised with 10 % (v/v) 0.2 M Tris.HCl buffer, not pH adjusted. In Fig. 2 a chromatogram is shown of the 280 nm signal of the collected fractions.

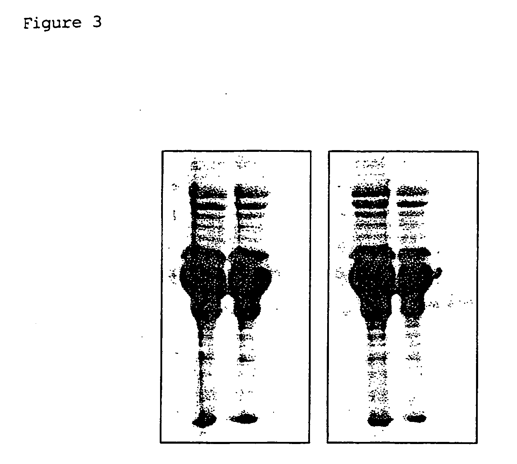

The eluted fractions were analyzed on Coomassie stained gel and western blot using anti-mouse Ig alkaline phosphatase conjugate for detection according to the instructions of the supplier (Promega) (see Fig. 3).

When using complete mouse serum for affinity purification a large fraction of the polyclonal antibodies are bound and eluted, whereas no contaminating protein such as serum albumin was found in the eluted fractions. Only a small proportion of antibodies was found in the non-bound fraction, which are probably derived from other isotypes of Ig (such as IgM, IgD or otherwise).

The results obtained with immobilised, tagged VHH and immobilised, untagged VHH are essentially the same, demonstrating that the provision of a peptide extension is not necessary to ensure that the immobilised VHH retains its antigen-binding capacity.

### EXAMPLE 5: Purification of Anti Freeze Peptide with VHH affinity columns.

After coupling of the anti-grass AFP VHH, a broth containing grass AFP was loaded onto the column. The buffers for the purification were, buffer A: 10 mM Na₂HPO₄/NaH₂PO₄, 150 mM NaCl pH 7.4, and elution buffer B 10 mM Na₂HPO₄/NaH₂PO₄, 150 mM NaCl with an addition of 12 mM HCl, final pH 2. Fraction collection was carried out manually; fractions were immediately neutralised with 10 % (v/v) 0.2 M Tris.HCl buffer, not pH adjusted. In Fig. 5 a chromatogram is shown of the 280 nm signal of the purification of Grass AFP with the untagged version of VHH#2. The identity of the eluted peak (Fraction 11) was checked using N-terminal amino acid sequence analysis. This was performed using the Edman degradation on a LF 3000 protein sequencer (Beckman) according to the supplier's protocol. The N-terminal sequence found was Asp-Glu-Gln-Pro-Asn-Thr-Ile-Ser-Gly-, in other words, the first nine residues of the N-terminus of the known sequence of AFP from *Lolium perenne*.

The untagged version of VHH#2 purified in the same way as the tagged version.

This example demonstrates that the absence of a peptide tail does not affect the capability of surface-bound VHH to bind antigen.

## Claims

1. An immunoactive material comprising a single domain antigen binding fragment of an immunoglobulin naturally devoid of light chains, or a protein which has the same or similar antigen binding properties located in a single domain, which fragment is devoid of any appended polypeptide group, immobilised by means of covalent bonds on a solid surface.

2. A material according to claim 1, wherein the single domain antigen-binding fragment is from a camelid immunoglobulin.

3. A material according to claim 1 or 2 wherein one or more amino acid residues in the framework region of the fragment is substituted by a different amino acid to facilitate immobilisation on the solid surface.

4. A material according to any one of claims 1 to 3, wherein the solid surface is selected from polystyrene, polypropylene, polyvinylchloride, polyacrylamide, celluloses, dextrans, synthetic polymers and co-polymers, latex, silica, agarose, metal, glass, carbon.

5. A material according to any one of claims 1 to 4 constituting all or part of a microtitre plate, a dipstick, a slide, a column or polymeric beads.

6. Use of a material according to any one of claims 1 to 5 in an immunoadsorption process.

7. Use according to claim 6 in an immunoassay or immunoaffinity purification process.

8. A method for immunoassay or purification comprising contacting a material according to any one of claims 1 to 5 with a test sample.

9. An array comprising a plurality of individual single domain antigen-binding fragments of an immunoglobulin naturally devoid of light chains, or a plurality of proteins which have the same or similar antigen binding properties located in a single domain, which fragments are devoid of any appended polypeptide group, immobilised on a solid surface, by means of covalent bonds.

## Patentansprüche

1. Mittels kovalenter Bindungen auf einer festen Fläche immobilisiertes, immunaktives Material, das ein Einzeldomänen-Antigenbindungsfragment eines Immunglobulins, dem leichte Ketten von Natur aus fehlen, oder ein Protein mit denselben oder ähnlichen Antigenbindungseigenschaften in einer Einzeldomäne aufweist, wobei an dem Fragment keine Polypeptidgruppe angehängt ist.

2. Material nach Anspruch 1, wobei das Einzeldomänen-Antigenbindungsfragment von einem Cameliden-Immunglobulinfragment stammt.

3. Material nach Anspruch 1 oder 2, wobei mindestens ein Aminosäurerest in der Hauptkettenregion des Fragments durch eine andere Aminosäure ersetzt ist, um die Immobilisierung auf der festen Fläche zu ermöglichen.

4. Material nach Anspruch 1 bis 3, wobei die feste Fläche aus Polystyrol, Polypropylen, Polyvinylchlorid, Polyacrylamid, Zellulosen, Dextranen, synthetischen Polymeren und Copolymeren, Latex, Silica, Agarose, Metall, Glas, Karbon ausgewählt ist.

5. Material nach einem der Ansprüche 1 bis 4, das aus einer ganzen Mikrotiterplatte, einem ganzen Eintauchstab (Dipstick), einem ganzen Objektträger, einer ganzen Säule oder ganzen Polymerkügelchen bzw. einem Teil davon besteht.

6. Verwendung eines Materials nach einem der Ansprüche 1 bis 5 in einem Immunadsorptionsprozess.

7. Verwendung nach Anspruch 6 in einem Immunassay oder einem Immunaffinitätsreinigungsprozess.

8. Verfahren für einen Immunassay oder eine Reinigung, welches das Inkontaktbringen eines Materials nach einem der Ansprüche 1 bis 5 mit einer Testprobe umfasst.

9. Mittels kovalenter Bindungen auf einer festen Fläche immobilisierte Anordnung (Array), welche mehrere einzelne Einzeldomänen-Antigenbindungsfragmenten eines Immunglobulins, dem leichte Ketten von Natur aus fehlen, oder mehrere Proteine mit denselben oder ähnlichen Antigenbindungseigenschaften in einer Einzeldomäne aufweist, wobei an den Fragmenten keine Polypeptidgruppe angehängt ist.

## Revendications

1. Matériau immunoréactif comprenant un fragment de liaison d'antigène à un seul domaine d'une immunoglobuline naturellement dépourvue de chaînes légères, ou une protéine qui a les mêmes propriétés de liaison d'antigène ou des propriétés de liaison d'antigène similaires situées dans un seul domaine, lequel fragment est dépourvu de tout groupe polypeptidique joint, immobilisé au moyen de liaisons covalentes sur une surface solide.

2. Matériau selon la revendication 1 où le fragment de liaison d'antigène à un seul domaine provient d'une immunoglobuline de camélidé.

3. Matériau selon la revendication 1 ou 2 où un ou plusieurs résidus d'aminoacides dans la région de charpente du fragment sont substitués par un aminoacide différent pour faciliter l'immobilisation sur la surface solide.

4. Matériau selon l'une quelconque des revendications 1 à 3 où la surface solide est choisie parmi le polystyrène, le polypropylène, le poly(chlorure de vinyle), le polyacrylamide, les celluloses, les dextranes, les polymères et copolymères synthétiques, le latex, la silice, l'agarose, un métal, le verre, le carbone.

5. Matériau selon l'une quelconque des revendications 1 à 4 constituant tout ou partie d'une microplaque de titrage, d'une bandelette réactive, d'une lamelle, d'une colonne ou de billes polymères.

6. Utilisation d'un matériau selon l'une quelconque des revendications 1 à 5 dans un procédé d'immunoadsorption.

7. Utilisation selon la revendication 6 dans une immunoanalyse ou un procédé de purification par immunoaffinité.

8. Procédé d'immunoanalyse ou de purification comprenant la mise en contact d'un matériau selon l'une quelconque des revendications 1 à 5 avec un échantillon test.

9. Matrice comprenant une pluralité de fragments de liaison d'antigène à un seul domaine individuels d'une immunoglobuline naturellement dépourvue de chaînes légères, ou une pluralité de protéines qui ont les mêmes propriétés de liaison d'antigène ou des propriétés de liaison d'antigène similaires situées dans un seul domaine, lesquels fragments sont dépourvus de tout groupe polypeptidique joint, immobilisés sur une surface solide, au moyen de liaisons covalentes.


### SEQUENCE LISTING

<110> Unilever plc
   Unilever NV
<120> Immobilisation of Protein
<130> T3082
<160> 4
<170> PatentIn version 3.0
<210> 1
   <211> 118
   <212> PRT
   <213> Lama
<400> 1
   Gln Val Gln Leu Gln Asp Ser Gly Gly Gly Leu Val Gln Ala Gly Gly
   1               5                   10                  15
   Ser Leu Arg Leu Ser Cys Ala Val Ser Gly Arg Thr Asp Ser Asn Tyr
               20                  25                  30
   Val Met Gly Trp Ser Arg Gln Ala Pro Gly Lys Gly Arg Glu Phe Ile
           35                  40                  45
   Ala Ala Ile His Trp Ser Glu Gly Gly Thr His Tyr Ala Asp Ser Val
       50                  55                  60
   Lys Asp Arg Phe Thr Ile Phe Arg Asp Ser Ala Lys Asn Ile Met Tyr
   65                  70                  75                  80
   Leu Gln Met Asn Gly Leu Lys Pro Glu Asp Thr Ala Val Tyr His Cys
                   85                  90                  95
   Ala His Asn Ser Gly Thr Gly Ala Phe Asp Tyr Trp Gly Gln Gly Thr
               100                 105                 110
   Gln Val Thr Val Ser Ser
           115
<210> 2
   <211> 128
   <212> PRT
   <213> Lama
<400> 2
   Gln Val Gln Leu Gln Glu Ser Gly Gly Gly Leu Val Glu Thr Gly Gly
   1               5                   10                  15
   Ser Leu Arg Leu Ser Cys Ala Ala Ser Gly Arg Thr Ile Ser Ser Tyr
               20                  25                  30
   Thr Ile Gly Trp Phe Arg Gln Ala Pro Gly Lys Glu Arg Glu Phe Val
           35                  40                  45
   Ser His His Phe Ala Ser Gly Gly Val Thr Asp Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Glu Met Asn Ser Leu Lys Pro Glu Asp Thr Ala Val Tyr Tyr Cys
                   85                  90                  95
   Ala Ala Ser Thr Phe Thr Ile Ser Gly Tyr Arg Ala Leu Lys Ala Ala
               100                 105                 110
   Tyr Glu Tyr Asp Tyr Trp Gly Gln Gly Thr Gln Val Thr Val Ser Ser
           115                 120                 125
<210> 3
   <211> 32
   <212> PRT
   <213> Artificial
<220>
   <223> Tag sequence for VHH
<400> 3
   Glu Gln Lys Leu Ile Ser Glu Glu Asp Leu Asn Gly Ala Ala Leu Arg
   1               5                   10                  15
   Ser Ile Phe Glu Ala Gln Lys Met Glu Trp His His His His His His
               20                  25                  30
<210> 4
   <211> 9
   <212> PRT
   <213> Lolium perenne
<400> 4
   Asp Glu Gln Pro Asn Thr Ile Ser Gly
   1               5